# EUROPÄISCHE PATENTANMELDUNG

(11) **EP 2 269 502 A1**
(43) Veröffentlichungstag der Anmeldung: **05.01.2011**
(21) Anmeldenummer: 10167746.6
(22) Anmeldetag: 29.06.2010
(51) Int. Cl.: A61B 5/022

(54) **Verfahren und Vorrichtung zur Bestimmung eines arteriellen Verschluss- und/oder Öffnungsdrucks in einem Blutgefäß**

(30) Priorität: 29.06.2009 DE 102009031172
(71) Anmelder: Enverdis GmbH, 07745 Jena (DE)
(72) Erfinder: Hübner, Thomas, 07749, Jena (DE); Alt, Michael, 36275, Kirchheim (DE)
(74) Vertreter: von Kreisler Selting Werner

(57) **Zusammenfassung**

Die Erfindung betrifft ein Verfahren zur Bestimmung eines arteriellen Verschluss- und/ oder Öffnungsdrucks in einem Blutgefäß. Hierbei wird eine Druckmanschette an einer Extremität eines Lebewesens appliziert. Der Manschettendruck wird auf einen Wert oberhalb des Verschlussdrucks erhöht. Der Verschlussdruck ist hierbei der Druck, bei dem der Blutfluss in dem Blutgefäß distal der Manschette zum Erliegen kommt, wobei der Manschettendruck kontinuierlich oder schrittweise gemessen wird. Die Erfindung betrifft ein Verfahren, bei dem aus dem Blutfüllungszustand der Extremität bzw. der Blutpulsation distal der Druckmanschette auf das Erreichen des Verschluss- bzw. Öffnungsdrucks geschlossen wird. Der Verschlussdruck gilt als erreicht, wenn der Maximalwert des Füllungszustandes der Extremität erreicht wird und/oder die Differenz der Form zwischen der erfassten Pulswelle und einer oder mehrerer regulärer Pulswellen einen festgelegten ersten Schwellwert überschreitet. Die Erfindung umfasst verschiedene Formparameter.

## Beschreibung

Die Erfindung betrifft ein Verfahren zur Bestimmung eines arteriellen Verschlussdrucks und/ oder Öffnungsdrucks in einem Blutgefäß.

Der sog. Ankle-Brachial-Index (ABI) ist eine einfache, nicht invasive Methode zur Diagnose einer peripheren arteriellen Verschlusskrankheit sowie zur Bestimmung des Herz-Kreislauf-Risikos.

Der ABI ist der Quotient aus den am Knöchel und am Oberarm eines Patienten gemessenen Werten des Verschluss- bzw. Öffnungsdrucks. Zur Bestimmung des ABI wird simultan oder nacheinander am Oberarm sowie im Knöchelbereich eine Druckmanschette appliziert. Hierbei wird schrittweise oder kontinuierlich der Manschettendruck auf Werte oberhalb des Druckes, bei welchem der Blutfluss distal der Manschette zum Erliegen kommt, erhöht und anschließend schrittweise bzw. kontinuierlich vermindert. Der Verschlussdruck ist der Manschettendruck, bei dem die Blutzirkulation distal der Manschette unterbrochen wird. Unter dem Öffnungsdruck versteht man den Manschettendruck, bei dem nach dem Ablassen des Drucks der suprasystolisch aufgepumpten Manschette die Blutzirkulation distal der Manschette wieder beginnt. Ein ABI-Wert von 0,9 bis 1,2 gilt als normal. Je kleiner der Quotient wird, desto größer ist das Ausmaß der Durchblutungsstörung. Werte unter 0,5 bedeuten meist eine klinisch relevante Ischämie mit sehr hoher Nekrose- und Ulkusgefahr. Werte von deutlich über 1,3 weisen auf eine besondere Art der Gefäßveränderung hin (Mediasklerose).

Die Messung wird an jedem Oberarm einmal und an den Knöcheln jeweils zweimal durchgeführt, da hier zwei Arterien existieren (Oberarm: Arteria brachialis, Knöchel: Arteria tibialis anterior und Arteria tibialis posterior). Zur Bildung des Quotienten wird der höhere der beiden Werte der letztgenannten Arterien verwendet. Alternativ zu zwei Messungen am Fuß des Patienten kann beispielsweise auch photoplethysmographisch der Blutfluss am Zeh des Patienten erfasst werden, wobei ein Blutfluss in den Digitalarterien im Zeh des Patienten dann vorliegt, wenn eine der beiden Arterien wieder blutdurchflossen ist.

Neben der Diagnosebestätigung und der Bestimmung des Schweregrades der peripheren arteriellen Verschlusskrankheit hat sich gezeigt, dass ein ABI von < 0,9 ein guter Parameter für die Vorhersage des Auftretens von späteren kardiovaskulären Komplikationen ist.

Gemäß dem Stand der Technik wird zur Berechnung des ABI beim liegenden Patienten beispielsweise mittels Doppler-Sonographie (mit einer Frequenz von 5 - 7 MHz) der Verschlussdruck am Oberarm und am Knöchel (hier zweifache Messung) gemessen. Die sonographische Verschlussdruckbestimmung ist nur schwer automatisierbar und birgt Fehlerquellen. Nur bei großer Erfahrung des Arztes ist mit einer geringen Fehlerrate von etwa 1% falschpositiven und 3% falschnegativen Ergebnissen zu rechnen.

Bei Diabetikern und Patienten mit chronischer Niereninsuffizienz können, bedingt durch das Vorliegen einer Mediasklerose, fälschlicherweise zu hohe (inkompressible) Doppler-Druckwerte (ABI > 1,3) auftreten. In diesem Fall wird eine Großzehen-Druckmessung durchgeführt. Der Verschlussdruck wird hierbei mittels Photoplethysmographie oder Laser-Doppler gemessen, da die Mediasklerose die Digitalarterien in der Regel nicht befällt. Dieser sog. Toe-Brachial-Index (TBI) liegt normalerweise bei Werten > 0,6.

Es ist ferner bekannt, den Verschlussdruck oszillographisch zu erfassen. Hierbei hat sich gezeigt, dass die so ermittelten Werte vielfach nicht mit den dopplersonographischen Werten übereinstimmen.

Eine weitere Schwierigkeit bei der Bestimmung des Verschluss- bzw. Öffnungsdruckes ist, dass kleine Blutpulsationen auch dann feststellbar sind, wenn der Manschettendruck deutlich oberhalb des systolischen Blutdrucks liegt. Somit ist es schwierig, den genauen Zeitpunkt festzustellen, zu dem der Blutfluss im Blutgefäß distal der Druckmanschette zum Erliegen kommt.

Aufgabe der Erfindung ist es, ein einfaches Verfahren sowie eine Vorrichtung zur genaueren Bestimmung des arteriellen Verschlussdrucks und/ oder Öffnungsdrucks in einem Blutgefäß bereitzustellen.

Diese Aufgabe wird erfindungsgemäß gelöst durch die Merkmale des Verfahrensanspruchs 1.

Das erfindungsgemäße Verfahren kann zur Bestimmung eines arteriellen Verschlussdrucks oder eines arteriellen Öffnungsdrucks oder beider Drücke in einem Blutgefäß verwendet werden und umfasst die folgenden Schritte:
Zunächst wird eine Druckmanschette an einer Extremität eines Lebewesens, vorzugsweise eines menschlichen Patienten, appliziert. Anschließend erfolgt ein Erhöhen des Manschettendrucks auf einen Wert oberhalb des Verschlussdrucks, nämlich des Drucks, bei dem der Blutfluss in dem Blutgefäß distal der Manschette zum Erliegen kommt. Hierbei wird der Manschettendruck kontinuierlich oder schrittweise gemessen. Das Erhöhen des Manschettendrucks kann ebenfalls kontinuierlich oder schrittweise erfolgen. Es erfolgt ein Ermitteln des Verschlussdrucks und/ oder des Öffnungsdrucks, indem mit Hilfe einer Detektionsvorrichtung detektiert wird, ob an der Extremität distal der Druckmanschette ein Blutfluss vorliegt. Die Frage, bei welchem genauen Druckwert ein Blutfluss vorliegt, d.h. zu welchem Zeitpunkt der Druckmanschettenerhöhung der Blutfluss zum Erliegen kommt bzw. zu welchem Zeitpunkt der Druckmanschettenverringerung der Blutfluss wieder beginnt, ist Gegenstand des erfindungsgemäßen Verfahrens.

Erfindungsgemäß erfolgt das Ermitteln des Verschlussdrucks und/ oder des Öffnungsdrucks durch folgende Schritte:
Zunächst werden der Verlauf der Pulswellen an der Extremität distal der Druckmanschette durch geeignete Mittel erfasst. Dies kann beispielsweise mittels Ultraschall, photoplethysmographisch, impendanzplethysmographisch, elektromagnetisch durch eine Induktionsspule, mittels Strain-Gauge, mit einem Dehnungsmessstreifen oder durch Messung der Druckschwankungen über eine weitere Manschette erfolgen. Geeignet ist jegliche Vorrichtung, die ein Erfassen des Pulswellenverlaufs ermöglicht.

Der erfasste Verlauf der Pulswellen kann gespeichert und durch eine Berechnungsvorrichtung, beispielsweise einen PC oder andere geeignete Hardware, weiter verarbeitet werden. Das Weiterverarbeiten der erfassen Pulswellenverläufe muss nicht zum gleichen Zeitpunkt wie die Erfassung des Verlaufs der Pulswellen stattfinden. Weiterhin kann sie an einem anderen Ort stattfinden und setzt nicht die Präsenz des menschlichen Körpers des Patienten voraus.

Im Wege der Verarbeitung der erfassten Pulswellen erfolgt ein Vergleichen der Amplitude und/ oder der Form und/ oder der Periodendauer der erfassten Pulswelle mit einer oder mehreren regulären Pulswellen, bei der oder bei denen der Verschlussdruck noch nicht erreicht war. Anfänglich kann beispielsweise als reguläre Pulswelle eine Pulswelle betrachtet werden, die bei einem unterhalb des systolischen Blutdrucks liegenden Manschettendruck erfasst wurde. Erfindungsgemäß gilt der Verschlussdruck dann als erreicht, wenn die Differenz der Amplitude und/ oder der Form und/ oder der Periodendauer zwischen der erfassten Pulswelle und einer oder mehrerer regulärer Pulswellen einen festgelegten ersten Schwellwert überschreitet. Mit anderen Worten, wird jede neu erfasste Pulswelle mit einem Wert verglichen, der sich aus einer oder mehreren regulären Pulswellen ableitet und der sich dynamisch verändern kann. Erfindungsgemäß wird daher nicht auf das Erreichen eines absoluten Wertes für die Amplitude, die Periodendauer oder anderer Parameter bezüglich der Form der erfassten Pulswelle abgestellt, sondern auf eine sprunghafte Veränderung in den genannten Werten. Sobald eine derartige sprunghafte Veränderung detektiert wird, d.h. eine ausreichend große Differenz registriert wurde, die den festgelegten ersten Schwellwert überschreitet, gilt der Verschlussdruck als erreicht.

In einer bevorzugten Ausführungsform erfolgt das Ermitteln des Verschlussdrucks und/ oder des Öffnungsdrucks durch folgende Schritte:
Zunächst wird eine Druckmanschette, deren Druck mittels geeigneter Mittel kontinuierlich gesteigert und gemessen wird, an der Extremität eines Lebewesens appliziert. Durch geeignete Mittel wird der Blutfluss bzw. Blutfüllungszustand der Extremität distal der Druckmanschette erfasst. Dies kann beispielsweise mittels Ultraschall, photoplethysmographisch, impedanzplethysmographisch, elektromagnetisch durch eine Induktionsspule, mittels Strain-Gauge-Plethysmographie, mit einem Dehnungsmessstreifen oder durch Messung der Druckschwankungen über eine weitere Manschette erfolgen. Geeignet ist jegliche Vorrichtung, die ein Erfassen der Blutfüllung des Gewebes bzw. des Pulswellenverlaufs ermöglicht. Ein Teil der genannten Methoden - wie die Erfassung von Bewegungen durch eine Induktionsspule im Magnetfeld oder das Ultraschall- bzw. Laser-Doppler-Verfahren - ist zur Messung insbesondere der Blutpulsationen geeignet. Andere Methoden wie eine hochauflösende Messung mit auf die Haut aufgeklebten Ultraschallsensoren, eine Umfangsmessung mit Dehnungsmessstreifen oder einer weiteren, mit einem Gas oder einer Flüssigkeit gefüllten Manschette, einer Strain-gauge- oder Photo-Plethysmographie erlauben die Erfassung sowohl des Blutfüllungszustandes als auch der Blutpulsationen.

Der erfasste Verlauf des Blutfüllungszustandes bzw. der Pulswellen kann gespeichert und durch eine Berechnungsvorrichtung, beispielsweise einen PC oder andere geeignete Hardware, weiter verarbeitet werden.

Im Wege der Verarbeitung der erfassten Messwerte erfolgt eine Beurteilung des Füllungszustandes der Extremitäten distal der Druckmanschette und/oder ein Vergleichen der erfassten Pulswelle mit einer oder mehreren regulären Pulswellen, bei der oder bei denen der Verschlussdruck noch nicht erreicht war.

Während der Druckzunahme der Druckmanschette werden bereits bei Druckwerten erheblich unterhalb des systolischen Blutdrucks die Venen verschlossen. Die Arterien dagegen sind aufgrund der höheren Blutdruckwerte gegenüber dem venösen Druck zunächst noch geöffnet. Damit fließt weiterhin Blut in die Extremität. Die Folge davon ist ein Blutstau in der Extremität distal der Druckmanschette. Dabei werden die Venen aufgrund ihrer Flexbilität geweitet.

Bei einem Druckanstieg von mindestens einigen mmHg pro Sekunde in der Druckmanschette wird der Verschlussdruck der Arterien erreicht, noch bevor die venöse Kapazität - der maximale Füllungszustand - voll ausgeschöpft wurde. D.h., dass der Blutfüllungszustand und damit der Umfang der Extremität bis zum Verschlussdruck ansteigen. Erst ab diesem Punkt, ab dem der weitere Bluteinstrom unterbrochen wird, kommt es - offenbar infolge der Penetration des Blutes durch die Gefäßwände und schließlich eines leichten Flüssigkeitsverlustes der Extremität distal der Druckmanschette - zu einem Abfall des Extremitätenumfangs. Damit fällt das Maximum des Umfangs der Extremität etwa mit dem Verschlussdruck der Arterien zusammen. Durch Messung des Extremitätenumfangs z.B. mit einer der oben beschriebenen mechanischen und elektrischen Methoden oder der Absorption einer auf die Hömoglobinkonzentration reagierenden Lichtwellenlänge kann damit der Verschlussdruck der Arterien bestimmt werden.

Solange die Arterien infolge eines über dem Verschlussdruck liegenden Manschettendrucks geschlossen sind, vermindert sich die Blutfüllung der Extremität distal der Manschette. Wird beim Ablassen des Manschettendrucks schließlich der Öffnungsdruck der Arterien unterschritten, strömt wiederum Blut in die Extremität ein. Dies führt wieder zu einem Anstieg der Blutfüllung der Extremität, damit zu einer Umfangssteigerung und Zunahme der Lichtabsorption. D.h. der Öffnungsdruck der Arterien fällt etwa mit einem relativen Minimum des Blutfüllungszustandes der Extremität zusammen. Der Öffnungsdruck ist damit ebenso wie der Verschlussdruck anhand des Verlaufs des Blutfüllungszustandes der Extremität distal der Druckmanschette detektierbar.

Der Vergleich der Amplitude und/ oder der Form der erfassten Pulswelle mit einer oder mehreren sogenannten regulären Pulswellen ist ein weiteres Kriterium zur Erfassung des Verschlussdrucks. Anfänglich kann beispielsweise als reguläre Pulswelle eine Pulswelle betrachtet werden, die bei einem unterhalb des systolischen Blutdrucks liegenden Manschettendruck erfasst wurde. Erfindungsgemäß gilt der Verschlussdruck dann als erreicht, wenn die Differenz der Amplitude und/ oder der Form zwischen der erfassten Pulswelle und einer oder mehreren regulären Pulswellen einen festgelegten ersten Schwellwert überschreitet. Mit anderen Worten wird jede neu erfasste Pulswelle mit einem Wert verglichen, der sich aus einer oder mehreren regulären Pulswellen ableitet und der sich dynamisch verändern kann. Erfindungsgemäß wird daher nicht auf das Erreichen eines absoluten Wertes für die Amplitude, die Periodendauer oder anderer Parameter bezüglich der Form der erfassten Pulswelle abgestellt, sondern auf eine rasche Veränderung in den genannten Werten. Sobald eine derartige rasche Veränderung detektiert wird, d.h. eine ausreichend große Differenz registriert wurde, die den festgelegten ersten Schwellwert überschreitet, gilt der Verschlussdruck als erreicht. So kann das Erreichen des Verschlussdrucks z.B. angenommen werden, wenn die Amplitude einer aktuell erfassten Pulswelle < 1/5, bevorzugt < 1/10 und besonders bevorzugt < 1/15 des Mittelwerts bisher erfasster regulärer Pulswellen ist.

Alternativ oder zusätzlich kann der Öffnungsdruck ermittelt werden. Dieser gilt dann als erreicht, wenn die Differenz der Amplitude und/ oder der Form zwischen der erfassten Pulswelle und einer oder mehrerer regulären Pulswellen einen festgelegten zweiten Schwellwert unterschreitet, der mit dem ersten Schwellwert identisch sein kann. Die Pulswellen, die unterhalb des ermittelten Verschlussdrucks erfasst wurden, können hierbei zu einem Referenzwert zusammengefasst werden, indem sie beispielsweise statistisch ausgewertet werden. Insbesondere kann der Mittelwert verschiedener Parameter aus diesen regulären Pulswellen gebildet werden. Mit diesem Mittelwert oder diesen Mittelwerten können die aktuell erfassten Pulswellen bei der Ermittlung des Öffnungsdrucks verglichen werden. Hierbei wird der suprasystolische Manschettendruck kontinuierlich oder schrittweise verringert, bis der Öffnungsdruck unter den genannten Voraussetzungen als erreicht gilt, d.h. bis wieder ein Blutfluss distal der Druckmanschette erfasst wird.

Bei der Ermittlung des Öffnungsdrucks werden daher die aktuell erfassten Pulswellen nicht mit einem sich dynamisch verändernden Wert verglichen sondern mit einem statischen Wert bzw. mit statischen Werten, die im Rahmen der Ermittlung des Verschlussdrucks festgelegt wurden. So ist es z.B. in der Praxis möglich, dass der Verschlussdruck bei einer bestimmten Amplitude, Form oder Periodendauer einer aktuell erfassten Pulswelle als erreicht gilt, aber der Öffnungsdruck, bei dem die gleichen Parameter (gleiche Amplitude und Form der Pulswelle) vorliegen, bei einem geringfügig anderen Druckwert erreicht wird. Aus diesem Grund ist es möglich, bei der Berechnung des ABI lediglich einen der genannten Druckwerte, beispielsweise nur den Öffnungsdruck, zu berücksichtigen, oder aber den Mittelwert aus dem Verschluss- und Öffnungsdruck.

Das erfindungsgemäße Verfahren bietet den Vorteil, dass, obwohl kleine Blutpulsationen auch dann feststellbar sein können, wenn der Manschettendruck deutlich oberhalb des systolischen Blutdrucks liegt, eindeutig der Verschlussdruck bzw. der Öffnungsdruck bestimmt werden kann. Es ist somit erfindungsgemäß möglich, den Übergangsbereich, in dem sich beispielsweise beim Erhöhen des Manschettendrucks die Pulswellen, ausgehend von ihrer ursprünglichen Form, Schritt für Schritt in Richtung eines Verlaufs ändern, der nicht mehr die Form einer Pulswelle aufweist, auszuwerten und innerhalb dieses Übergangsbereichs einen Punkt zu definieren, in dem der Verschlussdruck als erreicht gilt. Selbiges gilt für den Übergangsbereich bei der Manschettendruckverringerung im Rahmen der Ermittlung des Öffnungsdrucks.

Wenn die Differenz der Amplitude und/ oder der Form zwischen der erfassten Pulswelle und einer oder mehreren regulären Pulswellen einen festgelegten Schwellwert nicht überschreitet, kann die erfasste Pulswelle als reguläre Pulswelle betrachtet werden und der Gruppe der regulären Pulswellen zugerechnet werden. Hierbei können, wie bereits dargestellt, mehrere oder alle regulären Pulswellen statistisch ausgewertet werden, indem beispielsweise ein Mittelwert zum Vergleich mit der aktuell erfassten Pulswelle gebildet wird.

Neben einer regulären Pulswelle, die bei einem unterhalb des systolischen Blutdrucks liegenden Manschettendruck erfasst wurde, können weitere Pulswellen, bei denen die Differenz der Amplitude und/ oder der Form zu der oder den regulären Pulswellen geringer als ein festgelegter Schwellwert ist, der Gruppe der regulären Pulswellen zugerechnet werden. Diese Gruppe kann anschließend bei jedem Vergleich mit der nächsten aktuell erfassten Pulswelle statistisch ausgewertet werden, so dass die aktuell erfasste Pulswelle jeweils mit dem aktualisierten Mittelwert dieser Gruppe der regulären Pulswelle verglichen wird.

Vorzugsweise wird der Verlauf einer neuen Pulswelle während eines Zeitraums nach der letzen regulären Pulswelle erfasst, der der Länge von zwei oder bevorzugt drei Pulswellen entspricht. Hierbei wird vorzugsweise auf die Länge der bis dahin erfassten Pulswellen abgestellt. Hierbei kann es sich um einen Zeitraum von 2,5 sec. handeln. Die Betrachtung über diesen Zeitraum liegt darin begründet, dass vermieden werden soll, dass aufgrund von Herzarrhythmien und damit eines unregelmäßigen Verlaufs der erfassten Pulswelle der Verschlussdruck oder Öffnungsdruck fälschlicherweise als erreicht gilt. Beispielsweise können auch bei gesunden Menschen Extrasystolen auftreten. Diese können dazu führen, dass beispielsweise keine ausreichend hohe Amplitude einer Pulswelle erfasst wird. Um dies zu vermeiden wird daher ein etwas längerer Zeitraum betrachtet, der der Länge von zwei bis drei Pulswellen entspricht, so dass nach einer möglicherweise verfälschten Pulswelle eine oder zwei weitere Pulswellen betrachtet werden, in denen ggf. Werte für die Parameter, beispielsweise für die Amplitude, erfasst werden, die unterhalb des geforderten Schwellwertes liegen, so dass der Verschlussdruck nicht fälschlicherweise als erreicht gilt.

Bevorzugt ist der Verschlussdruck der Manschettendruck zum Zeitpunkt des der letzten regulären Pulswelle folgenden Minimums. Dies bedeutet, dass die erste nicht reguläre Pulswelle, d.h. die Pulswelle nach der letzten regulären Pulswelle, betrachtet wird, und in dieser Pulswelle das Minimum ermittelt wird, wobei der Manschettendruck zu dem Zeitpunkt dieses Minimums als Verschlussdruck angenommen wird. Insbesondere kann das Minimum über einen Zeitraum ermittelt werden, der der Länge von zwei oder bevorzugt drei Pulswellen entspricht oder während eines Zeitraums von 2,5 sec.

Zum Vergleich der Form der erfassten Pulswelle mit der oder den regulären Pulswellen können Parameter verwendet werden wie
- die zeitliche Distanz zwischen dem Fußpunkt und dem Wendepunkt der aufsteigenden Flanke der Pulswelle,
- Winkel zwischen der Senkrechten und der Tangenten im Wendepunkt der aufsteigenden Pulsflanke, wobei der Wendepunkt dem Maximum der ersten Ableitung entspricht.
- die zeitliche Distanz zwischen dem Fußpunkt und dem Maximum der Pulswelle,
- die zeitliche Distanz zwischen dem Wendepunkt der aufsteigenden Flanke und dem Maximum der Pulswelle
- ein Rundungsparameter, der durch den Radius eines in den Gipfel der Pulswelle gelegten Kreisbogens gekennzeichnet ist, wobei dieser lediglich um einen vorgegebenen Maximalbetrag vom Verlauf der Pulswelle abweicht und/oder
- die Periodendauer der Pulswelle.

Das Erreichen des Verschluss- bzw. des Öffnungsdrucks kann dann als erreicht angesehen werden, wenn einer oder mehrere der genannten Parameter einen festgelegten Schwellwert überschreiten. Das Erreichung des Verschlussdruckes könnte beispielsweise auch angenommen werden, wenn sich die Pulsanstiegszeit einer aktuell erfassten Pulswelle gegenüber dem Mittelwert der Pulsanstiegszeit der vorher registrierten regulären Pulswellen um mehr als das 1,15-fache, bevorzugt mehr als das 1,25-fache und besonders bevorzugt mehr als das 1,4-fache erhöht.

Zur Bestimmung des Rundungsparameters kann die Methode der kleinsten Fehlerquadrate zur Anpassung des Kreisbogens an den realen Kurvenverlauf im Maximum der Pulswelle angewandt werden, wobei der Kurvenverlauf beispielsweise oberhalb eines auf das Maximum bezogenen Prozentsatzes der Amplitude adaptiert wird.

Grundlage dieser Verfahrensschritte ist, dass es distal einer Gefäßstenose, wie sie beispielsweise durch die Druckmanschette verursacht wird, zu einer deutlichen Gestaltänderung der Pulswelle kommt, die einerseits durch eine Amplitudenabnahme, andererseits durch eine Verformung der Pulswelle gekennzeichnet ist. Unmittelbar nach einem durch eine Druckmanschette verursachten Verschluss eines Gefäßes wird beispielsweise kurzzeitig eine Erhöhung der Pulsperiodendauer gemessen. Hierbei gilt der Verschlussdruck als erreicht, wenn die Erhöhung der Periodendauer einen festgelegten Schwellwert überschreitet. Bei einer Verkürzung der Periodendauer unter einen bei der Ermittlung des Verschlussdrucks ermittelten Wert gilt dagegen der Öffnungsdruck als erreicht.

Die Bestimmung der Periodendauer einer Pulswelle kann - neben der Bestimmung des zeitlichen Abstandes zwischen markanten Punkten wie Maxima, Minima oder Wendepunkten der Pulswelle - mittels einer über ein geeignetes Zeitfenster durchgeführten Frequenzanalyse erfolgen, wobei dieses Zeitfenster kontinuierlich über die Pulswelle geschoben wird. Die Länge dieses Zeitfensters wird so gewählt, dass z.B. zwei Pulswellen (zwei Pulsperioden) erfasst werden. Zur Vermeidung von Effekten an den Rändern dieses Zeitfensters kann es sinnvoll sein, dieses mit einer in den Randbereichen verminderten Amplitudenfunktion (z.B, Hamming- oder Blackman-Fenster) zu belegen. Die Frequenzanalyse zeigt dann ein relatives Maximum im Bereich der Herzfrequenz (1/Pulsperiodendauer). Eine plötzliche Verschiebung dieses Frequenzmaximums kann ein Kriterium für das Erreichen des Verschlussdruckes darstellen.

Die Bestimmung des Verschlussdrucks oder des Öffnungsdrucks anhand der Pulsperiodendauer setzt einen hinreichend gleichmäßigen Puls voraus. Herzrhythmusstörungen können die Messgenauigkeit beeinflussen, beispielsweise wenn im Zeitfenster des erwarteten Gefäßverschlusses eine Extrasystole auftritt. Diesem Problem kann durch eine zeitgleiche Erfassung eines EKGs begegnet werden, wobei sich Herzrhythmusstörungen in einer veränderten elektrischen Herzerregung im EKG zeigen würden. Somit kann eine Abweichung der Pulsperiodendauer von der elektrokardiographisch bestimmten Periodendauer ein Kriterium für das Erreichen des Verschluss- bzw. Öffnungsdrucks darstellen.

Die Ursache einer plötzlichen Änderung der Pulsperiodendauer bei konstanter Herzfrequenz ist eine Änderung der Pulswellenlaufzeit. Dieser Parameter kann alternativ oder zusätzlich ebenfalls zur Feststellung des Erreichens des Verschlussdrucks und/ oder des Öffnungsdrucks herangezogen werden. Die genannte Feststellung wird belegt durch die bekannte Erhöhung der Pulswellenlaufzeiten bei pathologischen Gefäßverengungen. Eine plötzlich während der Manschettendruckerhöhung beobachtete Erhöhung der Pulswellenlaufzeit stellt damit ein Kriterium für das Erreichen des Verschlussdrucks dar. Umgekehrt ist eine Verminderung der Pulswellenlaufzeit während der Manschettendruckverringerung ein Kriterium für das Erreichen des Öffnungsdrucks. Beispielsweise ist es möglich, die Pulswellenlaufzeit dadurch zu bestimmen, dass die Zeitdifferenz zwischen markanten Punkten des EKG-Signals und der Pulswelle gemessen wird. Weiterhin kann eine Änderung der Pulswellenlaufzeit sensibel auch durch Messung der Phasenunterschiede zwischen der Pulswelle an der okkludierenden Druckmanschette und der Pulswelle distal der Druckmanschette erfasst werden. Hierzu muss selbstverständlich die Pulswelle an der Druckmanschette selbst durch geeignete Mittel erfasst werden.

Es ist bevorzugt, dass die statistische Auswertung, insbesondere die Mittelung der regulären Pulswellen, bis zum systolischen Blutdruck oder bis zum Erreichen des Verschlussdrucks durchgeführt werden. Der Mittelwert kann einzelne oder alle der genannten Parameter betreffen. Bei dem Erreichen des Verschlussdrucks können die bisher genannten Parameter herangezogen werden, so dass die statistische Auswertung solange durchgeführt wird, bis die genannten Parameter erreicht werden.

Als Amplitude einer Pulswelle wird beispielsweise der Abstand zwischen ihrem Fußpunkt und ihrem Maximum verstanden. Der Fußpunkt einer Pulswelle kann auf verschiedene Arten definiert werden. Eine Möglichkeit ist die Folgende: Zunächst wird eine Tangente durch den Punkt des steilsten Anstiegs der Pulswelle gelegt. Zu dieser Tangente wird eine Parallele ermittelt, die um einen festen Betrag von z. B. 10 ms vor der Tangente verläuft. Als Fußpunkt wird nun der Schnittpunkt der Parallele mit dem Verlauf der Pulswelle definiert.

Alternativ zur Definition der Amplitude auf Basis des Fußpunktes der Pulswelle kann z. B. auch das Minimum des Pulswellenverlaufs zwischen zwei Pulswellenmaxima verwendet werden.

Die Erfassung der Blutpulsationen distal der Druckmanschette kann - wie erwähnt - durch eine weitere Manschette erfolgen, wobei deren Druckpulsationen registriert werden. Zur Vereinfachung der Applikation ist jedoch auch eine mechanische Verbindung dieser Manschette mit der Druckmanschette möglich, so dass eine aus zwei Fluidkammern bestehende Manschette entsteht. Dabei kann die zur Messung dienende Fluidkammer über ein Druckminderungsventil mit der ersten Fluidkammer verbunden sein und damit einen vom Okklusionsdruck abhängigen, aber geringeren Druck aufweisen. Alternativ können die beiden Teile der Manschette, d.h. die beiden Fluidkammern, separat angesteuert und mit dem gewünschten Druck beaufschlagt werden.

Im Folgenden werden bevorzugte Ausführungsformen der Erfindung anhand von Figuren erläutert.

Es zeigen:
Fig. 1 eine schematisierte Darstellung einer Vorrichtung zur Durchführung des erfindungsgemäßen Verfahrens, und
Fig. 2 einen Ablaufplan zur Bestimmung des arteriellen Verschlussdruckes auf der Grundlage der Pulswellenanalyse distal der Druckmanschette.
Figuren 3a, 3b, 3c eine Darstellung des Zusammenhangs zwischen dem Blutfüllungszustand und dem Verschluss- und Öffnungsdruck.

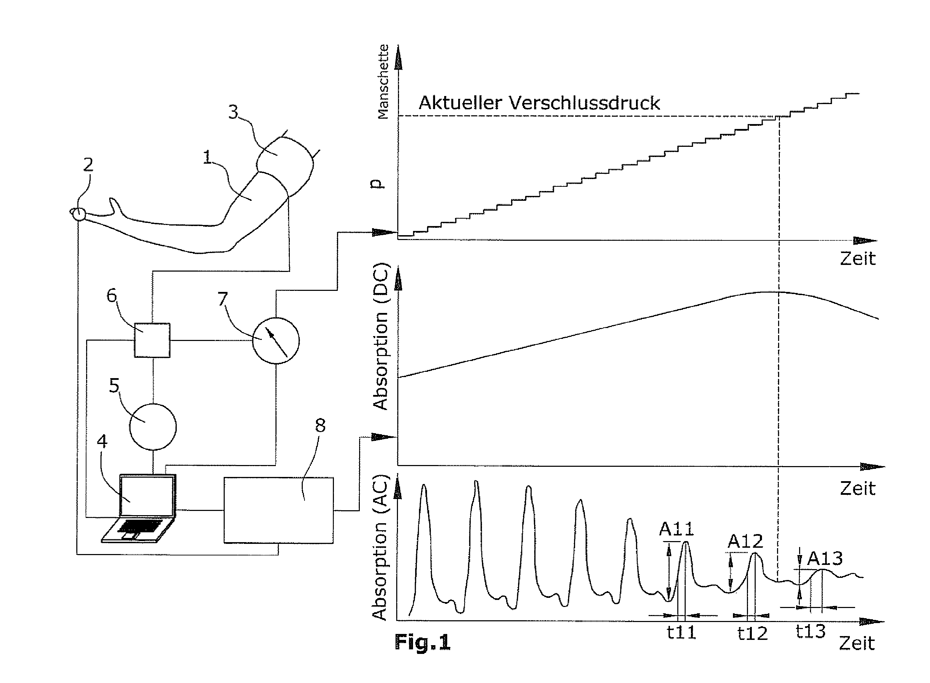

In Fig. 1 wird an einem Beispiel einer an einen Oberarm 1 eines Patienten angelegten Manschette 3 die erfindungsgemäße Verschlussdruckbestimmung dargestellt. Mittels einer Pumpe 5 wird der Manschettendruck in kleinen Schritten erhöht. Die Steuerung des Drucks erfolgt durch die Steuereinheit 4 in Verbindung mit einem Steuerventil 6 und einer Druckmessvorrichtung 7, die kontinuierlich den Manschettendruck misst. Ein plethysmographischer Sensor 2 registriert die Blutpulsationen, d.h. den Verlauf der Pulswellen, am Finger des Patienten und überträgt die Daten zum Plethysmographen 8.

Zur Ermittlung des Verschlussdrucks kann der Verlauf des Gleichanteils der Absorption (Absorption DC) und/oder der Verlauf des Wechselanteils der Absorption (Absorption AC) herangezogen werden.

Der gemessene Gleichanteil der Absorption, der sowohl in Transmission als auch in Reflexion mit einer auf die Hämoglobinkonzentration des Blutes reagierenden Wellenlänge erfasst werden kann, steigt während der Erhöhung des Manschettendrucks aufgrund der Stauwirkung infolge verschlossener Venen an. Dieser Anstieg der Absorption wird gestoppt, wenn die Arterien nach Erreichen des Verschlussdrucks verschlossen sind und kein weiteres Blut in die Extremität einfließen kann. Das Maximum der Absorptionskurve (Absorption DC) markiert damit den Verschlussdruck.

Darüber hinaus können aus dem Verlauf des Wechselanteils der Absorption (Absorption AC), der dem Gleichanteil überlagert ist, zusätzliche Informationen zur Bestimmung des Verschlussdrucks gewonnen werden. Dabei wird vorzugsweise der arithmetische Mittelwert der Amplituden A sowie der Mittelwert der Pulsanstiegszeiten t über alle regulären Pulswellen bis zur Pulswelle 12 (Amplitude A12) berechnet. Als Pulsanstiegszeit kann z.B. die Zeitspanne zwischen dem Punkt des steilsten Anstiegs der Pulswelle, d.h. dem Maximum der ersten Ableitung, und dem Maximum der Pulswelle verwendet werden. Zur Beurteilung der Pulswelle 13 wird die Amplitude A13 mit dem Mittelwert der Amplituden A1 - A12 verglichen. Ist A13 beispielsweise kleiner als 1/10 dieses Mittelwerts, so wird das Erreichen des Verschlussdrucks angenommen. In Fig. 1 ist dies noch nicht der Fall.

Allerdings hat sich die Form der Pulswelle 13 gegenüber dem Mittelwert der zwölf vorherigen Pulswellen verändert. Insbesondere hat sich die Pulsanstiegszeit t13 um mehr als das 1,3-fache erhöht. Dieser Parameter kann ebenfalls als Grenze für das Erreichen des Verschlussdrucks verwendet werden. Damit ist die Abweichung der Wellenform größer als das vorgegebene Maß. Die Pulswelle 13 wird als irregulär charakterisiert. Der Verschlussdruck gilt damit als erreicht.

Im vorliegenden Fall wurde definiert, dass der Verschlussdruck im Minimum vor der ersten irregulären Pulswelle (Amplitude A13) erreicht wird. Weiterhin ist es sinnvoll, nur einen begrenzten Zeitraum von zwei bis drei Pulswellen, d.h. etwa 2,5 sec. nach der letzten regulären Pulswelle (Amplitude A12) zu berücksichtigen.

Der Öffnungsdruck kann analog durch Erfassung der genannten Parameter zu Beginn des Bluteinstroms während des Öffnens der Arterien infolge einer Verringerung des Manschettendrucks bestimmt werden. Er wird beispielsweise zu dem Zeitpunkt angenommen, zu dem der Abfall des Gleichanteils der Absorptionskurve (Absorption DC) gestoppt wird die Kurve wieder ansteigt. Der Öffnungsdruck kann darüber hinaus aus dem Verlauf des Wechselanteils der Lichtabsorption (Absorption AC) ermittelt werden. So wird beispielsweise ein Mittelwert über die Werte für die Pulsanstiegszeit t1 bis t12 gebildet wird. Der Öffnungsdruck gilt dann als erreicht, wenn der Wert der aktuell erfassten Pulswelle für die Pulsanstiegszeit t kleiner als das 1,3-fache des Mittelwerts von t ist. Zusätzlich gilt der Öffnungsdruck ebenfalls als erreicht, wenn unabhängig von dem genannten Kriterium hinsichtlich der Pulsanstiegszeit die Amplitude der aktuell erfassten Pulswelle größer als 1/10 des Mittelwertes der Amplituden sämtlicher regulärerer Pulswellen, d.h. der Werte A1 bis A12, ist. Der Öffnungsdruck gilt dann als erreicht, wenn eines der beiden Kriterien erfüllt ist. Bei der Ermittlung des Öffnungsdrucks können auch andere Schwellwerte als bei der Ermittlung des Verschlussdrucks verwendet werden. Daher wurde in der vorliegenden Anmeldung der Begriff "erster Schwellwert" und "zweiter Schwellwert" verwendet, was nicht bedeutet, dass die beiden Schwellwerte verschieden sein müssen.

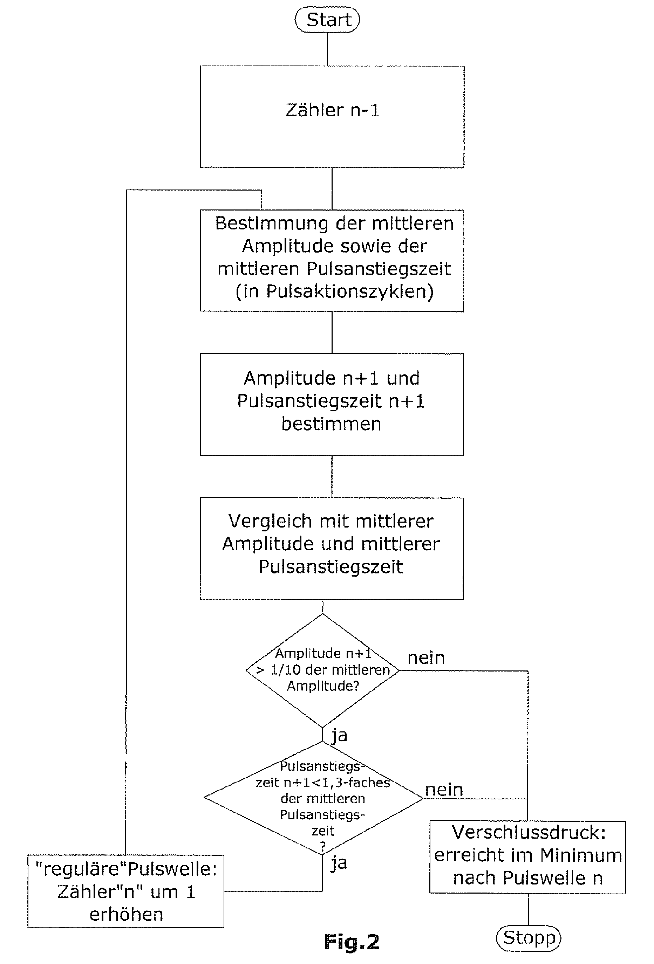

Fig. 2 stellt beispielhaft einen Ablaufplan zur Bestimmung des arteriellen Verschlussdrucks dar auf der Grundlage der Pulswellenanalyse distal der Druckmanschette dar. Es wird über mehrere Pulswellen die mittlere Amplitude A sowie die mittlere Pulsanstiegszeit t bestimmt. Bei jeder neuen Pulswelle wird die zugehörige Amplitude sowie die Pulsanstiegszeit berechnet und mit der mittleren Amplitude A und mittleren Pulsanstiegszeit t verglichen. Überschreitet die Abweichung festgelegte Grenzwerte, im vorliegenden Fall darf eine reguläre Pulswelle den Wert 1/10 der mittleren Amplitude A nicht unterschreiten oder das 1,3-fache der mittleren Pulsanstiegszeit t nicht überschreiten, wird das Erreichen des Verschlussdrucks angenommen. Andernfalls wird die neue erfasste Pulswelle als regulär angesehen und die zugehörige Amplitude bzw. Pulsanstiegszeit geht in die Mittelwerte dieser Parameter ein. Die Bewertung der folgenden Pulswellen beginnt wieder mit den ersten Schritten dieses Algorithmus.

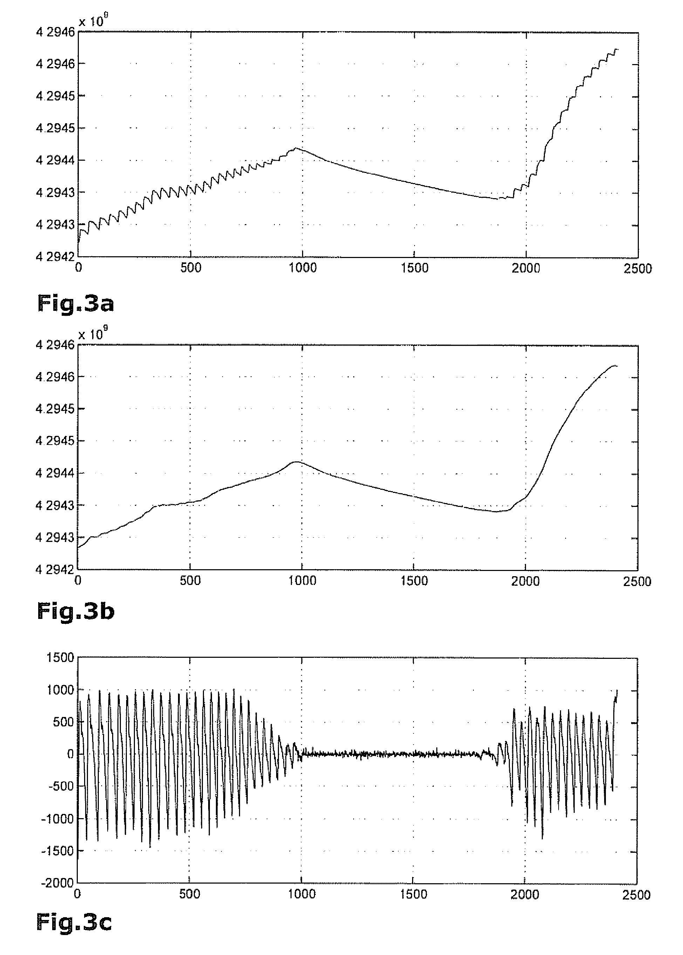

In Figur 3a ist der Verlauf der Blutfüllung einer Extremität distal einer Druckmanschette über die Zeit dargestellt. Hierbei ist sowohl der Gleich- als auch der Wechselanteil (Pulsationsanteil) sichtbar. Der Wechselanteil wird durch Pulsationen verursacht, die nach dem Erreichen des Verschlussdrucks (in etwa zum Zeitpunkt 1000) nicht mehr vorhanden sind und erst nach dem Erreichen des Öffnungsdrucks (kurz vor dem Zeitpunkt 2000) wieder beginnen.

In Figur 3b ist lediglich der Gleichanteil des Verlaufs des Blutfüllungszustandes über die Zeit dargestellt.

In Figur 3c ist der Wechselanteil aus Figur 3a dargestellt. Dieser kann über einen Hochpassfilter gefiltert werden. In Figur 3c wird besonders deutlich, dass nach dem Erreichen des Verschlussdrucks und vor dem Erreichen des Öffnungsdrucks keine wesentlichen Blutpulsationen an der Extremität distal der Druckmanschette messbar sind. Das lokale Maximum aus Figur 3b ungefähr beim Zeitpunkt 1000 sowie das lokale Minimum kurz vor dem Zeitpunkt 2000 fallen somit mit dem Erreichen des Verschlussdrucks bzw. des Öffnungsdrucks in Figur 3c zusammen.

## Patentansprüche

1. Verfahren zur Bestimmung eines arteriellen Verschluss- und/ oder Öffnungsdrucks in einem Blutgefäß mit den Schritten:
- Applizieren einer Druckmanschette an einer Extremität eines Lebewesens,
- Kontinuierliches oder schrittweises Erhöhen des Manschettendrucks von einem Wert unterhalb des Verschlussdrucks auf einen Wert oberhalb des Verschlussdrucks, nämlich des Drucks, bei dem der Blutfluss in dem Blutgefäß distal der Manschette zum Erliegen kommt, wobei der Manschettendruck gemessen wird,
- Erfassen des Verlaufs der Blutfüllung der Extremität distal der Druckmanschette,
- Ermitteln des Verschlussdrucks und/ oder des Öffnungsdrucks, anhand des Verlaufs der Blutfüllung,
**dadurch gekennzeichnet, dass**
das Ermitteln des Verschlussdrucks und/ oder des Öffnungsdrucks folgende Schritte umfasst:
- Erfassen des Pulsationsverlaufs bzw. des Wechselanteils des Blutfüllungszustandes, wobei die Form einer erfassten Pulswelle mit einer oder mehreren regulären Pulswellen, bei der oder bei denen der Verschlussdruck noch nicht erreicht war, verglichen wird,
- wobei der Verschlussdruck dann als erreicht gilt, wenn die Differenz der Amplitude und/ oder der Form und/ oder der Periodendauer zwischen der erfassten Pulswelle und einer oder mehrerer regulärer Pulswellen einen festgelegten ersten Schwellwert überschreitet und/ oder der Öffnungsdruck dann als erreicht gilt, wenn diese Differenz einen festgelegten zweiten Schwellwert unterschreitet.

2. Verfahren nach Anspruch 1, **dadurch gekennzeichnet, dass**, wenn die Differenz der Amplitude und/ oder der Form zwischen der erfassten Pulswelle und einer oder mehrerer regulärer Pulswellen einen festgelegten Schwellwert nicht überschreitet, die erfasste Pulswelle als reguläre Pulswelle betrachtet wird oder der Gruppe der regulären Pulswellen zugerechnet wird, wobei insbesondere mehrere oder alle regulären Pulswellen statistisch ausgewertet werden, indem vorzugsweise ein Mittelwert zum Vergleich mit der aktuell erfassten Pulswelle gebildet wird.

3. Verfahren nach Anspruch 1 oder 2, **dadurch gekennzeichnet, dass** die Form einer erfassten Pulswelle mit einer mehreren regulären Pulswellen hinsichtlich eines oder mehrerer der Parameter
- zeitliche Distanz zwischen dem Fußpunkt und dem Wendepunkt der aufsteigenden Flanke der Pulswelle,
- Winkel zwischen der Senkrechten und der Tangente im Wendepunkt der aufsteigenden Pulsflanke,
- zeitliche Distanz zwischen dem Fußpunkt und dem Maximum der Pulswelle,
- zeitliche Distanz zwischen dem Wendepunkt der aufsteigenden Flanke und dem Maximum der Pulswelle
- Rundungsmaß, das durch den Radius eines in den Gipfel der Pulswelle gelegten Kreisbogens **gekennzeichnet** ist und dieser Kreisbogen lediglich um einen vorgegebenen Maximalbetrag vom Verlauf der Pulswelle abweicht,
- Periodendauer der Pulswelle,
verglichen wird und der Verschlussdruck dann als erreicht gilt, wenn die Differenz der Form zwischen der erfassten Pulswelle und einer oder mehrerer regulärer Pulswellen einen festgelegten ersten Schwellwert überschreitet und/ oder der Öffnungsdruck dann als erreicht gilt, wenn die Differenz der Form zwischen der erfassten Pulswelle und einer oder mehrerer regulärer Pulswellen einen festgelegten zweiten Schwellwert unterschreitet.

4. Verfahren nach einem der Ansprüche 1 bis 3, **dadurch gekennzeichnet, dass** zur Bestimmung des Rundungsparameters die Methode der kleinsten Fehlerquadrate bei der Anpassung des Kreisbogens an den realen Kurvenverlauf im Maximum der Pulswelle angewandt wird.

5. Verfahren nach einem der Ansprüche 1 bis 4, **dadurch gekennzeichnet, dass** als reguläre Pulswelle eine Pulswelle betrachtet wird, die bei einem unterhalb des systolischen Blutdrucks liegenden Manschettendruck erfasst wurde, und weitere Pulswellen, bei denen die Differenz der Form zu der oder den regulären Pulswellen geringer als ein festgelegter Schwellwert ist, der Gruppe der regulären Pulswellen zugerechnet werden.

6. Verfahren nach einem der Ansprüche 1 bis 5, **dadurch gekennzeichnet, dass** der Vergleich der erfassten Pulswelle mit dem Mittelwert aus mehreren regulären Pulswellen erfolgt.

7. Verfahren nach einem der Ansprüche 1 bis 6, **dadurch gekennzeichnet, dass** die Detektionsvorrichtung distal der Druckmanschette auf optischer, elektromagnetischer oder akustischer Basis beruht.

8. Verfahren nach einem der Ansprüche 1 bis 6, **dadurch gekennzeichnet, dass** die Detektionsvorrichtung distal der Druckmanschette auf mechanischer Basis beruht, wobei die Umfangsmessungen der Extremitäten entweder mittels strain-gauge-Plethysmographie oder einer weiteren, mit einem Gas oder einer Flüssigkeit gefüllten Mess-Manschette besteht.

9. Verfahren nach Anspruch 8, **dadurch gekennzeichnet, dass** die Mess-Manschette mit der Druckmanschette mechanisch verbunden ist, aber einen unterschiedlichen inneren Druck aufweist.

10. Verfahren zur Bestimmung eines arteriellen Verschluss- und/ oder Öffnungsdrucks in einem Blutgefäß mit den Schritten:
- Applizieren einer Druckmanschette an einer Extremität eines Lebewesens,
- Kontinuierliches oder schrittweises Erhöhen des Manschettendrucks von einem Wert unterhalb des Verschlussdrucks auf einen Wert oberhalb des Verschlussdrucks, nämlich des Drucks, bei dem der Blutfluss in dem Blutgefäß distal der Manschette zum Erliegen kommt, sofern der Verschlussdruck bestimmt werden soll oder kontinuierliches oder schrittweises Verringern des Manschettendrucks von einem Wert oberhalb des Verschlussdrucks auf einen Wert unterhalb des Verschlussdrucks, sofern der Öffnungsdruck bestimmt werden soll, wobei der Manschettendruck beim Erhöhen oder Verringern des Manschettendrucks gemessen wird,
- Ermitteln des Verschlussdrucks und/ oder des Öffnungsdrucks, indem mit Hilfe einer Detektionsvorrichtung detektiert wird, ob an der Extremität distal der Druckmanschette ein Blutfluss vorliegt,
**dadurch gekennzeichnet, dass**
das Ermitteln des Verschlussdrucks und/ oder des Öffnungsdrucks folgende Schritte umfasst:
- Erfassen des Verlaufs der Blutfüllung der Extremität distal der Druckmanschette,
- Bestimmen des Manschettendrucks zu dem Zeitpunkt an dem der Verlauf der Blutfüllung sein Maximum erreicht hat, sofern der Verschlussdruck bestimmt werden soll, oder zu dem Zeitpunkt an dem der Verlauf der Blutfüllung ein lokales Minimum im Bereich des systolischen Blutdrucks erreicht hat, sofern der Öffnungsdruck bestimmt werden soll, wobei der Manschettendruck zum Zeitpunkt des Maximums dem Verschlussdruck und der Manschettendruck zum Zeitpunkt des lokalen Minimums dem Öffnungsdruck entspricht.
